# EUROPÄISCHE PATENTANMELDUNG

(11) **EP 4 846 960 A1**
(43) Veröffentlichungstag der Anmeldung: **02.12.2026**
(21) Anmeldenummer: 26178586.9
(22) Anmeldetag: 13.05.2026
(51) Int. Cl.: A61B 17/80, B33Y 80/00

(54) **IMPLANTAT ZUR REKONSTRUKTION EINES KNOCHENDEFEKTS**

(30) Priorität: 27.05.2025 DE 102025120636
(71) Anmelder: Karl Leibinger Asset Management GmbH & Co. KG, 78570 Mühlheim an der Donau (DE)
(72) Erfinder: Goodson, Alexander MC, Warren Farm, Longwood, Winchester SO21 1LA (GB); Hollmann, Frederik, 69117 Heidelberg (DE)
(74) Vertreter: Schwamberger, Martin

(57) **Zusammenfassung**

Implantat (S) zur Rekonstruktion eines Knochendefekts, wobei das Implantat (S) eine erste Osteosyntheseplattenanordnung (O1) zur Verbindung eines ersten Knochensegments (K1) mit einem zweiten Knochensegment (K2) sowie eine zweite Osteosyntheseplattenanordnung (O2) zur Verbindung des zweiten Knochensegments (K2) mit einem dritten Knochensegment (K3) aufweist, wobei die erste und zweite Osteosyntheseplattenanordnung (O1, O2) genau eine oder genau zwei Osteosyntheseplatten (O1a, O1b, O2a, O2b) aufweist, wobei die Osteosyntheseplatten (O1a, O1b, O2a, O2b) eine stangenartige Form mit einem ersten Längsende und einem gegenüberliegenden zweiten Längsende aufweisen, wobei die Osteosyntheseplatte oder -platten (O1a, O1b) der ersten Osteosyntheseplattenanordnung (O1) und die Osteosyntheseplatte oder -platten (O2a, O2b) der zweiten Osteosyntheseplattenanordnung (O2) mit einem Verbindungselement (V) unmittelbar verbunden sind, wobei das Verbindungselement (V) räumlich zwischen der ersten und zweiten Osteosyntheseplattenanordnung (O1, O2) angeordnet ist, und wobei das Verbindungselement (V) derart ausgestaltet ist, dass das Verbindungselement (V) die Osteosyntheseplatten (O1a, O1b, O2a, O2b) an ihrem dem Verbindungselement (V) zugewandten Längsende verbindet.

## Beschreibung

Die vorliegende Erfindung betrifft ein Implantat zur Rekonstruktion eines Knochendefekts, welche insbesondere aufgrund Verlust oder Vorschädigung eines menschlichen oder tierischen Knochenanteils vorliegt, beispielsweise eines Unterkieferknochens.

Es bestehen verschiedene Ursachen, welche zum teilweisen oder vollständigen Verlust von Unterkieferanteilen führen können. Beispielsweise kann die Knochenstruktur in diesem Bereich durch ein Trauma schwer beschädigt sein. Im Falle eines Tumors in diesem Bereich ist häufig eine Entfernung oder Bestrahlung der lokalen Knochenstruktur erforderlich. Durch die Strahlentherapie kann die Knochenstruktur beschädigt werden, sodass eine Verankerung einer Unterkiefer-Zahnprothese an der verbleibenden lokalen Struktur unzuverlässig ist. Auch systemische Faktoren wie eine Chemotherapie, eine Immuntherapie oder die Einnahme von Knochenstoffwechsel-relevanter Medikamente können zur Schädigung dieses Bereichs führen.

Zur Behandlung von Patienten mit einer derartigen Diagnose ist es bekannt, den entstandenen Knochendefekt zu rekonstruieren, beispielsweise durch Auffüllen mit einem Spenderknochen. Im Falle einer Unterkiefer-Rekonstruktion ist beispielsweise bekannt, die beschädigten Unterkieferteile zu entfernen und einzelne Segmente aus der Fibula zu entnehmen, neu zu orientieren, und in der neuen Orientierung am verbleibenden Unterkieferanteil zu befestigen, wie beispielsweise in der US 2020222060 A1 beschrieben. Zur Ausrichtung der Fibula-Segmente in der gewünschten Position sowie zur Befestigung am verbleibenden Unterkiefer sind ein oder mehrere Implantate erforderlich, insbesondere Osteosyntheseplatten.

Die US 2022168028 A1 beschreibt mehrere Lösungen für ein derartiges Rekonstruktions-Implantat. In Fig. 1 der US 2022168028 A1 wird eine einzige Osteosyntheseplatte beschrieben, welche insgesamt vier Knochensegmente miteinander verbindet und derart einen Knochendefekt rekonstruiert. Diese Lösung wird jedoch als nachteilig angesehen, da im Falle einer lokalen Infektion die Osteosyntheseplatte und gegebenenfalls die zur Rekonstruktion verwendeten Knochensegmente entfernt werden müssen. Zudem ist eine derartige Konstruktion verhältnismäßig steif, sodass zur Heilung förderliche Mikrobewegungen zwischen den Knochensegmenten begrenzt sind.

In Fig. 2 der US 2022168028 A1 wird eine weitere Lösung für ein derartiges Rekonstruktions-Implantat beschrieben. Bei dieser Lösung werden benachbarte Knochensegmente durch je eine einzelne Osteosyntheseplatte miteinander verbunden. Um eine korrekte Ausrichtung der einzelnen Osteosyntheseplatten zu gewährleisten, wird ein Hilfsrahmen vorgeschlagen, welcher die einzelnen Osteosyntheseplatten miteinander verbindet und in der gewünschten Relativposition hält. Nach erfolgreicher Anbringung der Osteosyntheseplatten wird der Hilfsrahmen entfernt. Die Osteosyntheseplatten werden zusammen mit dem Hilfsrahmen durch additive Fertigung erzeugt, beispielsweise aus Titan oder aus einem biokompatiblen Polymer.

Aus der US 20220142687 A1 ist eine Lösung bekannt, bei der eine Fraktur oder ein Spalt im Unterkiefer durch zwei parallel angeordnete Osteosyntheseplatten überbrückt wird. Soll eine oder mehrere derartige Lösungen mit einem Hilfsrahmen gemäß Fig. 2 der US 2022168028 A1 in Position gehalten werden, so ist ein sehr filigraner und aufwändiger Hilfsrahmen erforderlich.

Es ist Aufgabe der Erfindung, die Lösung gemäß Fig. 2 der US 2022168028 A1 weiterzuentwickeln, sodass eine zuverlässige Fertigung ohne Funktionseinschränkungen ermöglicht wird.

Die Aufgabe wird gelöst durch die Merkmale des Patentanspruchs 1. Vorteilhafte Ausgestaltungen ergeben sich aus den abhängigen Patentansprüchen, der Beschreibung sowie aus den Figuren.

Zur Lösung wird ein Implantat zur Rekonstruktion eines Knochendefekts vorgeschlagen. Das Implantat weist zumindest eine erste Osteosyntheseplattenanordnung und eine zweite Osteosyntheseplattenanordnung auf. Die erste Osteosyntheseplattenanordnung dient zur Verbindung eines ersten Knochensegments mit einem zweiten Knochensegment. Die zweite Osteosyntheseplattenanordnung dient zur Verbindung des zweiten Knochensegments mit einem dritten Knochensegment. Durch das zweite Knochensegment kann somit ein Knochendefekt zwischen dem ersten und dritten Knochensegment rekonstruiert werden.

Die erste Osteosyntheseplattenanordnung weist genau eine oder genau zwei Osteosyntheseplatten auf. Die zweite Osteosyntheseplattenanordnung weist ebenfalls genau eine oder genau zwei Osteosyntheseplatten auf. Die Osteosyntheseplatten weisen eine stangenartige Form mit einem ersten Längsende und einem gegenüberliegenden zweiten Längsende auf. Unter einer stangenartigen Form wird hierbei eine längliche Form verstanden, die sich geradlinig oder gekrümmt zwischen ihren beiden Längsenden erstreckt. Die Osteosyntheseplatten sind derart angeordnet, dass sie einen Spalt zwischen den ihnen zugeordneten Knochensegmenten überbrücken, sodass der Spalt jeweils zwischen dem ersten Längsende und dem zweiten Längsende der jeweiligen Osteosyntheseplatte angeordnet ist.

Die Osteosyntheseplatte oder -platten der ersten Osteosyntheseplattenanordnung und die Osteosyntheseplatte oder -platten der zweiten Osteosyntheseplattenanordnung sind mit einem Verbindungselement unmittelbar verbunden. Unter einer unmittelbaren Verbindung wird hierbei eine Verbindung ohne Zwischenelemente verstanden, beispielsweise in Form einer einteiligen Ausgestaltung von Osteosyntheseplatten und Verbindungselement, oder in Form einer Steckverbindung.

Das Verbindungselement ist räumlich zwischen der ersten und zweiten Osteosyntheseplattenanordnung angeordnet und derart ausgestaltet, dass das Verbindungselement die Osteosyntheseplatten an ihrem dem Verbindungselement zugewandten Längsende verbindet.

Durch einen derartigen Aufbau des Implantats wird eine im wesentlichen längliche Struktur geschaffen, die keine oder nur wenige räumlich überlappende Elemente aufweist. Eine derartige Struktur ist in einem additiven Fertigungsverfahren prozesssicher herstellbar.

Vorzugsweise weist die erste und/oder die zweite Osteosyntheseplattenanordnung jeweils genau zwei Osteosyntheseplatten auf, welche parallel zueinander angeordnet sind. In anderen Worten wird die erste und/oder die zweite Osteosyntheseplattenanordnung durch je ein Paar von Osteosyntheseplatten gebildet. Der der Osteosyntheseplattenanordnung zugeordnete Spalt wird somit an zwei Stellen durch je eine Osteosyntheseplatte überbrückt. Durch eine derartige Ausgestaltung können die einzelnen Osteosyntheseplatten dünnwandiger hergestellt werden, da sich die Belastung auf zwei Osteosyntheseplatten verteilt. Dadurch werden die zur Heilung des Knochenspalts förderlichen Mikrobewegungen erhöht. Die vorgeschlagene Ausbildung des Verbindungselements ist für eine derartige Gestaltung der Osteosyntheseplattenanordnungen besonders einfach anwendbar, da die längliche Gesamtstruktur des Implantats einen lokal oder durchgehenden parallel angeordneten Aufbau erhält.

Sind die erste und/oder die zweite Osteosyntheseplattenanordnung durch je ein Paar von Osteosyntheseplatten gebildet, so ist dieses Paar von Osteosyntheseplatten vorzugsweise ausschließlich über das Verbindungselement miteinander verbunden. **In** anderen Worten sind bei einer derartigen Ausgestaltung neben dem Verbindungselement vorzugsweise keine weiteren Hilfselemente vorgesehen, welche das jeweilige Paar von Osteosyntheseplatten zueinander in Position hält.

Vorzugsweise weist das Verbindungelement zumindest einen nach lateral nach außen gewölbten Bogen auf. Unter lateral nach außen wird dabei eine Richtung verstanden, die von der Körpermitte des Patienten wegweist, wenn das Implantat in seiner Soll-Orientierung am Patienten angeordnet ist. Der Bogen verbindet zwei der Osteosyntheseplatten miteinander, wobei die somit verbundenen Osteosyntheseplatten jeweils unterschiedlichen Osteosyntheseplattenanordnungen zugeordnet sind. Durch diese Bogenform liegt das Verbindungselement zumindest abschnittsweise nicht am Knochen an, wenn das Implantat in seiner Soll-Orientierung am Patienten angeordnet ist. Dadurch kann das auf dem Knochensegment in diesem Bereich vorliegenden Weichgewebe besser geschützt werden. So werden beispielsweise das Periosteum und Muskeln im Bereich des Verbindungselements nicht beschädigt, sodass die Blutversorgung des Knochensegments sichergestellt werden kann.

Vorzugsweise weist der zumindest eine Bogen einen inhomogenen Querschnitt auf, wobei der kleinste Querschnitt des Bogens an einem Kontaktbereich zu den Osteosyntheseplatten angeordnet ist. Dadurch kann das Verbindungselement in diesem Kontaktbereich einfacher von den Osteosyntheseplatten getrennt werden, nachdem das Implantat erfolgreich am Patienten befestigt worden ist. Die Abtrennung des Verbindungselements kann beispielsweise durch eine chirurgische Zange erfolgen. Durch das lateral nach außen gewölbte Verbindungselement kann der Chirurg das Verbindungselement dabei umgreifen, sodass das Verbindungselement sicher entfernt werden kann, ohne das Knochensegment oder das darauf befindliche Weichgewebe zu beschädigen.

Die Querschnittsreduktion kann auch als Sollbruchstelle ausgebildet sein, sodass ein werkzeugloses Abtrennen des Verbindungselements erfolgen kann, nachdem das Implantat erfolgreich am Patienten befestigt worden ist.

Sind die erste und zweite Osteosyntheseplattenanordnung jeweils durch ein Osteosyntheseplatten-Paar gebildet, so weist das Verbindungselement vorzugsweise genau zwei lateral nach außen gewölbte Bögen auf, nämlich einen ersten Bogen und einen zweiten Bogen. Der erste Bogen verbindet eine der Osteosyntheseplatten der ersten Osteosyntheseplattenanordnung mit einer der Osteosyntheseplatten der zweiten Osteosyntheseplattenanordnung, und der zweite Bogen verbindet die andere Osteosyntheseplatte der ersten Osteosyntheseplattenanordnung mit der anderen Osteosyntheseplatte der zweiten Osteosyntheseplattenanordnung.

Weist das Verbindungselement genau zwei lateral nach außen gewölbte Bögen auf, so sind die beiden Bögen vorzugsweise durch eine Verbindungsstrebe miteinander verbunden. Dadurch wird auf einfache Weise eine Relativpositionierung zwischen den beteiligten Osteosyntheseplatten gewährleistet.

Vorzugsweise weist die Verbindungsstrebe einen lateral hervorstehenden Vorsprung auf, beispielsweise in Form einer ringförmigen Öse. Dadurch kann das Implantat während dem Implantationsvorgang durch ein geeignetes Instrument besser gehalten werden. Vorzugsweise ist der Vorsprung an der Verbindungsstrebe ausgebildet.

Die vorgeschlagene Lösung ist nicht auf ein Implantat zur Verbindung und Ausrichtung von drei Knochensegmenten beschränkt. So kann das Implantat beispielsweise auch eine dritte Osteosyntheseplattenanordnung aufweisen, um das dritte Knochensegment mit einem vierten Knochensegment zu verbinden. Die dritte Osteosyntheseplattenanordnung ist dabei in gleicher Weise aufgebaut wie die erste und/oder zweite Osteosyntheseplattenanordnung. Zur Verbindung der zweiten Osteosyntheseplattenanordnung mit der dritten Osteosyntheseplattenanordnung ist ein weiteres Verbindungselement vorgesehen, welches räumlich zwischen der zweiten und dritten Osteosyntheseplattenanordnung angeordnet ist.

Das weitere Verbindungselement ist derart ausgestaltet, dass das weitere Verbindungselement die ihr zugeordneten Osteosyntheseplatten an ihrem dem weiteren Verbindungselement zugewandten Längsende verbindet. Das weitere Verbindungselement kann dabei so gestaltet werden wie das Verbindungselement zwischen der ersten und zweiten Osteosyntheseplattenanordnung. In gleicher Weise könnte das Implantat auch eine vierte oder fünfte Osteosyntheseplattenanordnung aufweisen

Vorzugsweise ist das Implantat einteilig ausgeführt und in einem additiven Verfahren gefertigt, beispielsweise in einem 3D-Druck-Verfahren. Beispiele für geeignete Fertigungsverfahren sind Selektives Laserschmelzen oder Selektives Lasersintern. Das Implantat ist vorzugsweise aus Titan oder einem biokompatiblen Polymer hergestellt. Auch die Nutzung von bioresorbierbaren Werkstoffen ist denkbar.

Vorzugsweise handelt es sich bei dem Implantat um ein Unterkiefer-Rekonstruktions-Implantat oder um ein Oberkiefer-Rekonstruktions-Implantat. Das Implantat ist vorzugsweise patientenspezifisch geplant und geformt, sodass ein passgenaues Anliegen des Implantats an den jeweiligen Patienten gewährleistet ist.

Die im Rahmen der vorliegenden Erfindung vorgeschlagene Konstruktion ist nicht auf einen Einsatz am Oberkiefer oder Unterkiefer beschränkt. Das vorgeschlagene Implantat kann an verschiedenen Stellen am menschlichen oder tierischen Körper zum Einsatz kommen, besonders wenn mehrere kleine Osteosyntheseplatten in einer zueinander definierten Lage zum Einsatz kommen sollen. Ein mögliches weiteres Anwendungsbeispiel ist ein Orthognathie-Implantat zur Repositionierung eines Teils der Maxilla. Ein weiteres Anwendungsbeispiel ist eine Jochbein-Osteotomie zur Neugestaltung des Jochbeins, um die ästhetische Form des Gesichts zu verbessern oder um eine anatomische Fehlstellung zu korrigieren. Ein weiteres Anwendungsbeispiel ist eine Neupositionierung der Schädeldecken-Teile bei Kleinkindern, um eine anatomische Fehlstellung zu korrigieren. Ein weiteres Anwendungsbeispiel ist das simultane Einsetzen mehrerer Zahnimplantate in entsprechende Fehlstellen. Ein weiteres Anwendungsbeispiel ist ein Einsatz im Bereich der Wirbelsäule.

Ausführungsbeispiele der Erfindung sind anhand der Figuren detailliert beschrieben. Es zeigen:
- Fig. 1 bis Fig. 6: zeigen je eine Ansicht eines Implantats gemäß einem ersten Ausführungsbeispiel;
- Fig. 7: zeigt eine Ansicht eines Implantats gemäß einem zweiten Ausführungsbeispiel;
- Fig. 8 bis Fig. 10: zeigen je eine Ansicht eines Implantats gemäß einem dritten Ausführungsbeispiel; und
- Fig. 11: zeigt eine Detailansicht eines Verbindungselements.

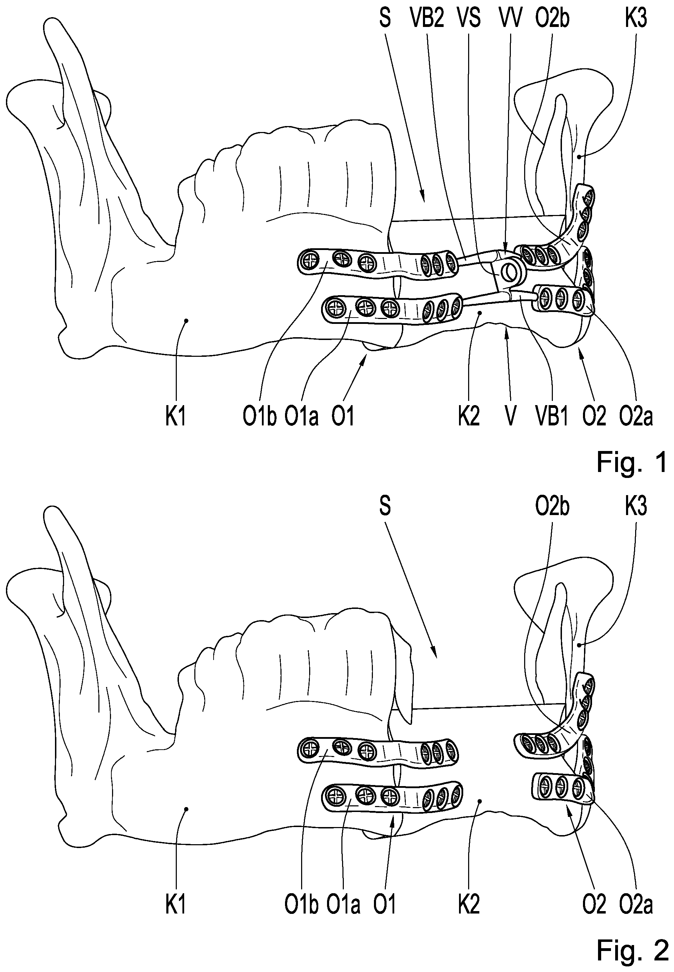

Fig. 1 zeigt eine Ansicht eines Implantats S gemäß einem ersten Ausführungsbeispiel. Das Implantat S dient zur Rekonstruktion einer Knochendefekts eines Unterkieferknochens. Der Unterkieferknochen weist ein Knochensegment K1 und ein Knochensegment K3 auf. Zwischen dem Knochensegment K1 und dem Knochensegment K3 liegt eine Fehlstelle vor, welche durch ein Knochensegment K2 aufgefüllt, bzw. rekonstruiert werden soll. Das Knochensegment K2 ist im vorliegenden Beispiel aus einer Scapula des Patienten entnommen. Die Knochensegmente K1 und K3 wurden zuvor durch Resektion auf ein geeignetes Maß gebracht, um das Knochensegment K2 aufzunehmen zu können.

Das Implantat S dient zur Befestigung des Knochensegments K2 an den Knochensegmenten K1 und K3. Zur Befestigung dienen hierzu mehrere Osteosyntheseplatten O1a, O1b, O2a, O2b. Um einen exakten Sitz des Knochensegments K2 in der Fehlstelle zu gewährleisten, sind die Osteosyntheseplatten O1a, O1b, O2a, O2b vorab geplant und entsprechend dieser Planung patientenspezifisch gefertigt. Die Osteosyntheseplatten O1a, O1b sind einer ersten Osteosyntheseplattenanordnung O1 zugeordnet, und verbinden das Knochensegment K1 mit dem Knochensegment K2. Die Osteosyntheseplatten O2a, O2b sind einer zweiten Osteosyntheseplattenanordnung O2 zugeordnet, und verbinden das Knochensegment K2 mit dem Knochensegment K3. Die Knochensegmente K1, K2, K3 sind somit durch zwei Paare von Osteosyntheseplatten O1a, O1b, O2a, O2b verbunden, wobei die Osteosyntheseplatten O1a, O1b; O2a, O2b eines derartigen Paares im Wesentlichen parallel zueinander angeordnet sind. Die Osteosyntheseplatten O1a, O1b, O2a, O2b weisen je eine stangenartige, der knöchernen Struktur folgende Form auf, und erstrecken sich länglich zwischen einem ersten Längsende und einem zweiten Längsende. Die Osteosyntheseplatten O1a, O1b, O2a, O2b weisen mehrere Löcher auf, welcher zur Hindurchführung von Befestigungsschrauben dienen. Mittels den Befestigungsschrauben können die Osteosyntheseplatten O1a, O1b, O2a, O2b mit den ihnen zugeordneten Knochensegmenten K1, K2, K3 verbunden werden.

Zwischen den beiden Osteosyntheseplattenanordnungen O1 und O2 ist ein Verbindungselement V vorgesehen. Das Verbindungselement V weist eine untere bogenförmige Strebe VB1 auf, welche die Osteosyntheseplatte O1a mit der Osteosyntheseplatte O2a verbindet. Das Verbindungselement V weist ferner eine obere bogenförmige Strebe VB2 auf, welche die Osteosyntheseplatte O1b mit der Osteosyntheseplatte O2b verbindet. Die beiden bogenförmigen Streben VB1, VB2 sind durch eine vertikal ausgerichtete Verbindungsstrebe VS miteinander verbunden. Durch das Verbindungselement V ist die Position der Osteosyntheseplatten O1a, O1b, O2a, O2b vor dem Implantationsvorgang vordefiniert, sodass dem Chirurgen die Ausrichtung des Implantats S und dessen Befestigung an den Knochensegmenten K1, K2, K3 erleichtert wird.

Fig. 2 zeigt eine weitere Ansicht des Implantats S gemäß dem ersten Ausführungsbeispiel. In der Darstellung gemäß Fig. 2 ist das Implantat S bereits erfolgreich am Patienten befestigt worden, sodass der Knochendefekt zwischen den Knochensegmenten K1 und K3 durch das Knochensegment K2 aufgefüllt, bzw. rekonstruiert wurde. In diesem Zustand wird das Verbindungselement V nicht mehr benötigt, sodass es durch den Chirurgen entfernt wurde.

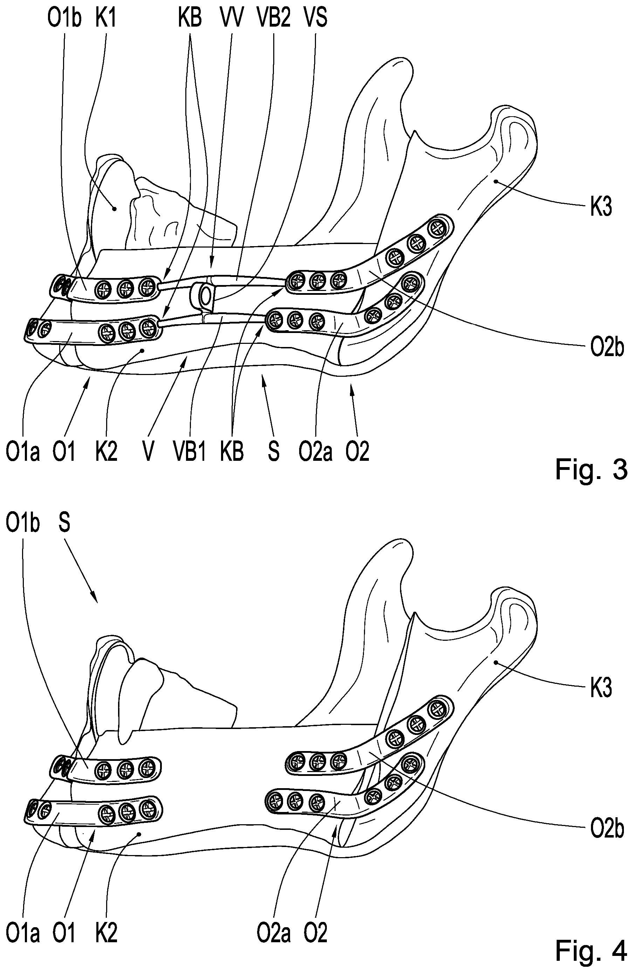

Fig. 3 zeigt eine weitere Ansicht des Implantats S gemäß dem ersten Ausführungsbeispiel. In der Darstellung gemäß Fig. 3 ist das Verbindungselement V noch nicht entfernt worden. In Fig. 3 sind die Kontaktbereiche KB hervorgehoben, an denen das Verbindungselement V in die Osteosyntheseplatten O1a, O1b, O2a, O2b übergeht. Hierbei ist deutlich zu erkennen, dass das Verbindungselement V jeweils an eines der Längsenden der Osteosyntheseplatten O1a, O1b, O2a, O2b anschließt. Ferner ist deutlich zu erkennen, dass an der Verbindungsstrebe VS ein lateral nach außen gerichteter Vorsprung VV angeordnet ist, welcher als ringförmige Öse ausgebildet ist. An diesem Vorsprung VV kann das Implantat S mit einem Instrument gehalten, und am Patienten ausgerichtet werden. Soll nun das Verbindungselement V entfernt werden, so kann der Chirurg das Verbindungselement V mit am Vorsprung VV fixieren und die Streben VB1, VB2 an den Kontaktbereichen KB zu den Osteosyntheseplatten O1a, O1b, O2a, O2b mit einer Zange durchtrennen.

Fig. 4 zeigt eine weitere Ansicht des Implantats S gemäß dem ersten Ausführungsbeispiel. Die in Fig. 4 dargestellte Ansicht entspricht der in Fig. 3 dargestellten Ansicht, wobei jedoch das Verbindungselement V von den Osteosyntheseplatten O1a, O1b, O2a, O2b entfernt worden ist.

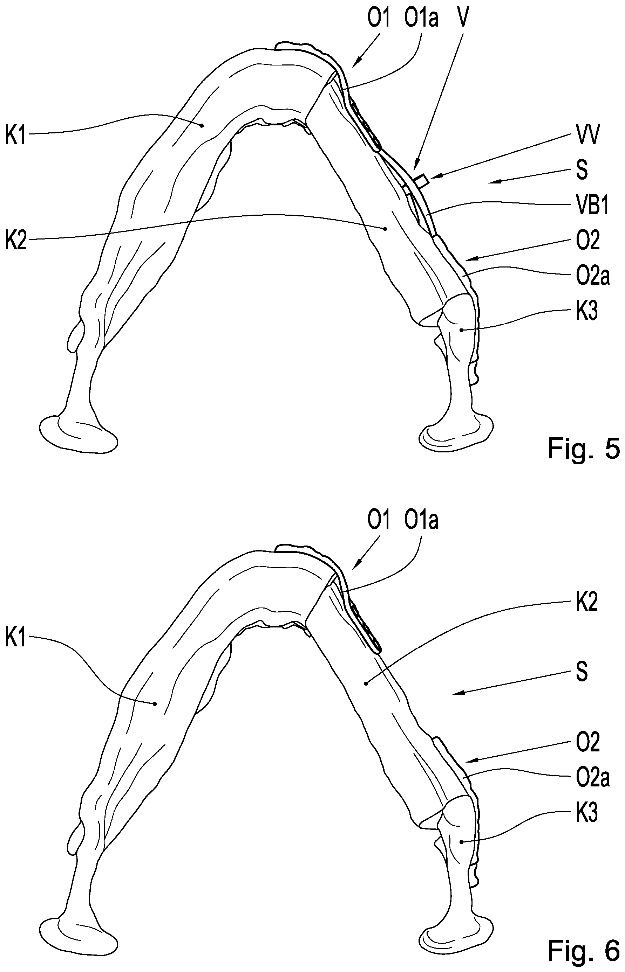

Fig. 5 zeigt eine weitere Ansicht des Implantats S gemäß dem ersten Ausführungsbeispiel, wobei der Unterkiefer von unten dargestellt ist. In dieser Ansicht ist der Knochendefekt zwischen den Knochensegmenten K1 und K3, sowie das Auffüllen dieser Fehlstelle durch das Knochensegment K2 deutlich zu erkennen. Darüber hinaus ist in Fig. 5 die bogenförmige Gestaltung der Strebe VB1 des Verbindungselements V deutlich zu erkennen. Die bogenförmige Strebe VB1 ist deutlich nach lateral außen gewölbt, sodass der in der Mitte des Bogens angeordnete Vorsprung VV vom Knochensegment K2 entfernt ist. Ferner geht aus der Darstellung in Fig. 5 deutlich hervor, dass die bogenförmige Strebe VB1 einen inhomogenen Querschnitt aufweist, wobei der kleinste Querschnitt am Übergang des Bogens zu den Osteosyntheseplatten O1a, O2a angeordnet ist. Durch diese Gestaltung wird das Entfernen der Streben VB1, VB2 von den Osteosyntheseplatten O1a, O1b, O2a, O2b erleichtert.

Fig. 6 zeigt eine weitere Ansicht des Implantats S gemäß dem ersten Ausführungsbeispiel. Die in Fig. 6 dargestellte Ansicht entspricht der in Fig. 5 dargestellten Ansicht, wobei das Verbindungselement V von den Osteosyntheseplatten O1a, O1b, O2a, O2b entfernt worden ist.

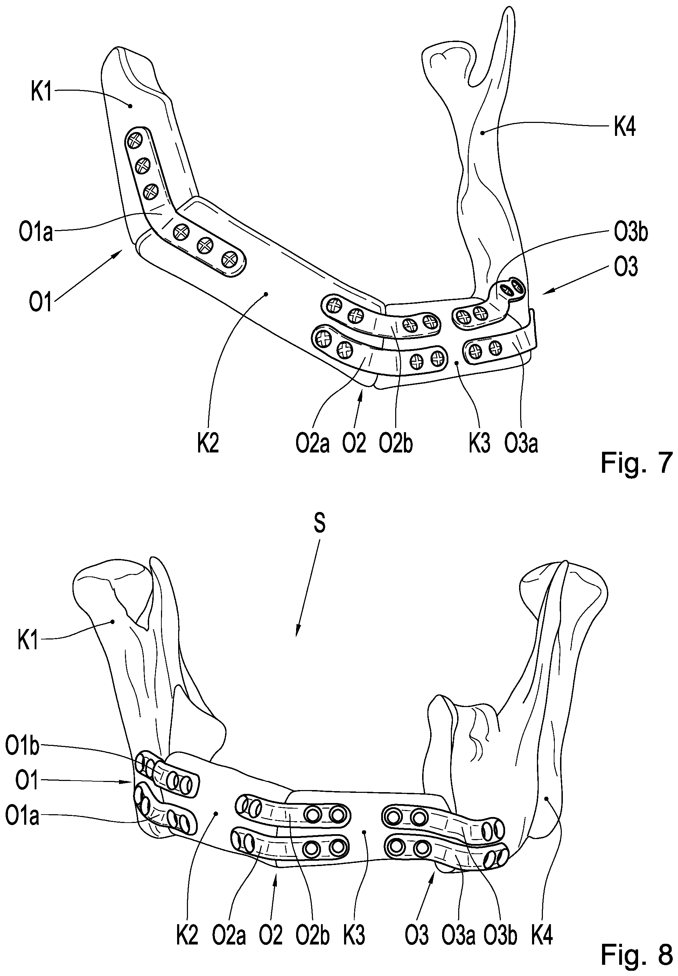

Fig. 7 zeigt eine Ansicht eines Implantats S gemäß einem zweiten Ausführungsbeispiel. Das Implantat S gemäß dem zweiten Ausführungsbeispiel ist zur Befestigung und Ausrichtung von zwei Knochensegmenten K2, K3 eingerichtet, um einen Knochendefekt zwischen dem Knochensegment K1 und einem Knochensegment K4 eines Unterkieferknochens aufzufüllen, bzw. zu rekonstruieren. Dazu weist das Implantat S eine erste Osteosyntheseplattenanordnung O1 auf, welche im vorliegenden Beispiel nur aus einer Osteosyntheseplatte O1a besteht, um das Knochensegment K1 mit dem Knochensegment K2 zu verbinden. Darüber hinaus weist das Implantat S eine zweite Osteosyntheseplattenanordnung O2 auf, welche aus zwei Osteosyntheseplatten O2a, O2b besteht, um das Knochensegment K2 mit dem Knochensegment K3 zu verbinden. Schließlich weist das Implantat S eine dritte Osteosyntheseplattenanordnung O3 auf, welche aus zwei Osteosyntheseplatten O3a, O3b besteht, um das Knochensegment K3 mit dem Knochensegment K4 zu verbinden.

In der Darstellung gemäß Fig. 7 sind die Verbindungselemente zur Verbindung der ersten und zweiten Osteosyntheseplattenanordnung O1, O2 sowie zur Verbindung der zweiten und dritten Osteosyntheseplattenanordnung O2, O3 nicht dargestellt. Das Verbindungselement zur Verbindung der zweiten und dritten Osteosyntheseplattenanordnung O2, O3 entspricht im Wesentlichem dem Aufbau des Verbindungselements V wie in Fig. 1 bis 6 dargestellt.

Das Verbindungselement zur Verbindung der ersten und zweiten Osteosyntheseplattenanordnung O1, O2 entspricht im Wesentlichen ebenfalls dem Aufbau des Verbindungselements V wie in Fig. 1 bis 6 dargestellt, allerdings nur mit einer Verbindungsstrebe zwischen der Osteosyntheseplatte O1a und der Verbindungsstrebe VS.

Fig. 8 zeigt eine Ansicht eines Implantats S gemäß einem dritten Ausführungsbeispiel. Ähnlich wie in dem in Fig. 7 dargestellten zweiten Ausführungsbeispiel liegt ein Knochendefekt zwischen dem Knochensegment K1 und dem Knochensegment K4 eines Unterkieferknochens vor, welche durch genau zwei aus der Fibula entnommene Knochensegmente K2, K3 aufgefüllt, bzw. rekonstruiert werden soll. Dazu sind insgesamt drei Osteosyntheseplattenanordnungen O1, O2, O3 vorgesehen, welche jeweils ein Paar Osteosyntheseplatten O1a, O1b; O2a, O2b; O3a, O3b aufweisen. In der Darstellung gemäß Fig. 8 sind die Verbindungselemente zur Verbindung der ersten und zweiten Osteosyntheseplattenanordnung O1, O2 sowie zur Verbindung der zweiten und dritten Osteosyntheseplattenanordnung O2, O3 nicht dargestellt.

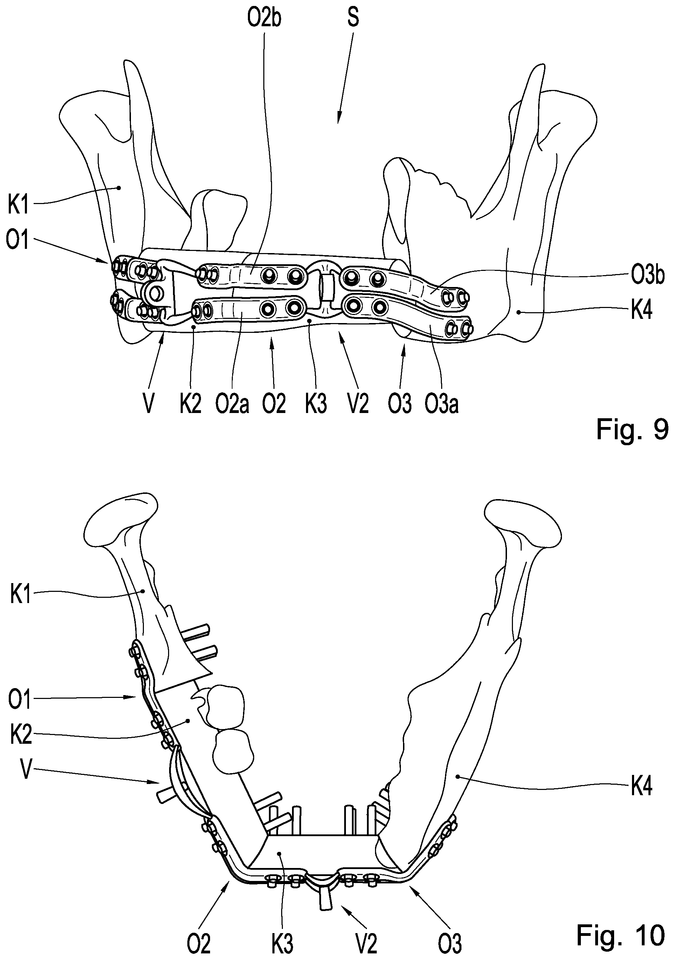

Fig. 9 und Fig. 10 zeigen je eine weitere Ansicht des Implantats S gemäß dem dritten Ausführungsbeispiel. In dieser Ansicht ist das Verbindungselement V zur Verbindung der ersten und zweiten Osteosyntheseplattenanordnung O1, O2 sowie das Verbindungselement V2 zur Verbindung der zweiten und dritten Osteosyntheseplattenanordnung O2, O3 dargestellt. Besonders aus der Darstellung gemäß Fig. 10 geht deutlich hervor, dass die Verbindungselemente V, V2 den grundsätzlich gleichen Aufbau aufweisen, wobei die exakte Form des Bogens sowie die Länge der bogenartigen Verbindungsstreben von der jeweiligen Distanz zwischen den Osteosyntheseplattenanordnungen O1, O2 bzw. O2, O3 abhängig sind. Anstelle der Befestigungsschrauben sind in Fig. 9 und Fig. 10 die Vektoren der Befestigungsschrauben dargestellt.

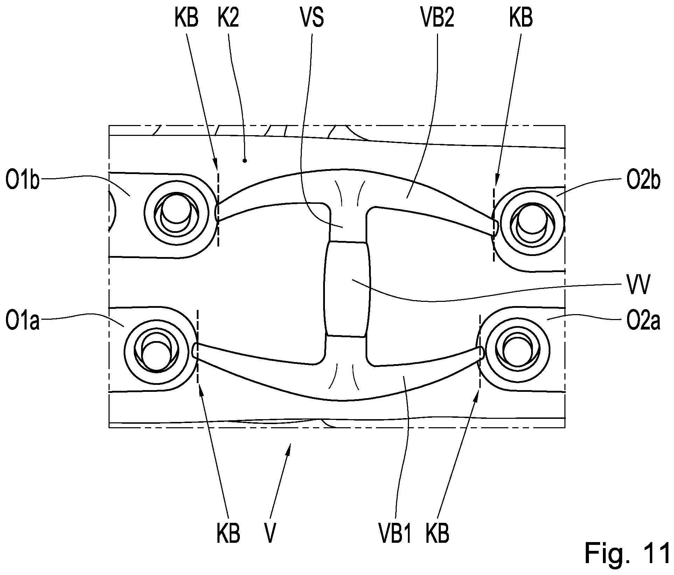

Fig. 11 zeigt eine Detailansicht des Verbindungselements V sowie des Kontaktbereichs KB des Verbindungselements V zu den Osteosyntheseplatten O1a, O1b, O2a, O2b. Aus der Darstellung in Fig. 11 geht der inhomogene Querschnitt der bogenförmigen Streben VB1, VB2 deutlich hervor. Der kleinste Querschnitt ist an den Kontaktbereichen KB angeordnet, um das Entfernen des Verbindungselements V von den Osteosyntheseplatten O1a, O1b, O2a, O2b zu vereinfachen.

Die in den Figuren dargestellten Implantate S stellen allesamt Beispiele für ein Unterkiefer-Rekonstruktions-Implantat dar. Die Erfindung ist jedoch in keiner Weise auf die Anwendung am Unterkiefer beschränkt. Beispielsweise ist das vorgeschlagene Implantat in gleicher Gestaltung auch für eine Anwendung am Oberkiefer geeignet, um einen Knochendefekt des Oberkiefers zu rekonstruieren.

### Bezugszeichenliste

- S: Implantat
- O1: erste Osteosyntheseplattenanordnung
- O1a: Osteosyntheseplatte
- O1b: Osteosyntheseplatte
- O2: zweite Osteosyntheseplattenanordnung
- O2a: Osteosyntheseplatte
- O2b: Osteosyntheseplatte
- O3: dritte Osteosyntheseplattenanordnung
- O3a: Osteosyntheseplatte
- O3b: Osteosyntheseplatte
- V: Verbindungselement
- V2: Weiteres Verbindungselement
- VB1: Erster Bogen
- VB2: Zweiter Bogen
- VS: Verbindungstrebe
- VV: Vorsprung
- KB: Kontaktbereich
- K1: Knochensegment
- K2: Knochensegment
- K3: Knochensegment
- K4: Knochensegment

## Patentansprüche

1. Implantat (S) zur Rekonstruktion eines Knochendefekts,
- wobei das Implantat (S) eine erste Osteosyntheseplattenanordnung (O1) zur Verbindung eines ersten Knochensegments (K1) mit einem zweiten Knochensegment (K2) sowie eine zweite Osteosyntheseplattenanordnung (O2) zur Verbindung des zweiten Knochensegments (K2) mit einem dritten Knochensegment (K3) aufweist,
- wobei die erste Osteosyntheseplattenanordnung (O1) genau eine oder genau zwei Osteosyntheseplatten (O1a, O1b) aufweist, und wobei die zweite Osteosyntheseplattenanordnung (O2) genau eine oder genau zwei Osteosyntheseplatten (O2a, O2b) aufweist,
- wobei die Osteosyntheseplatten (O1a, O1b, O2a, O2b) eine stangenartige Form mit einem ersten Längsende und einem gegenüberliegenden zweiten Längsende aufweisen,
- wobei die Osteosyntheseplatte oder -platten (O1a, O1b) der ersten Osteosyntheseplattenanordnung (O1) und die Osteosyntheseplatte oder -platten (O2a, O2b) der zweiten Osteosyntheseplattenanordnung (O2) mit einem Verbindungselement (V) unmittelbar verbunden sind,
- wobei das Verbindungselement (V) räumlich zwischen der ersten und zweiten Osteosyntheseplattenanordnung (O1, O2) angeordnet ist, und
- wobei das Verbindungselement (V) derart ausgestaltet ist, dass das Verbindungselement (V) die Osteosyntheseplatten (O1a, O1b, O2a, O2b) an ihrem dem Verbindungselement (V) zugewandten Längsende verbindet.

2. Implantat (S) nach Anspruch 1, **dadurch gekennzeichnet, dass** die erste und/oder die zweite Osteosyntheseplattenanordnung (O1, O2) jeweils genau zwei Osteosyntheseplatten (O1a, O1b; O2a, O2b) aufweist, welche parallel zueinander angeordnet sind.

3. Implantat (S) nach Anspruch 2, **dadurch gekennzeichnet, dass** ein Osteosyntheseplatten-Paar (O1a, O1b; O2a, O2b) der Osteosyntheseplattenanordnung (O1, O2) ausschließlich über das Verbindungselement (V) miteinander verbunden ist.

4. Implantat (S) nach einem der Ansprüche 1 bis 3, **dadurch gekennzeichnet, dass** das Verbindungselement (V) zumindest einen lateral nach außen gewölbten Bogen (VB1, VB2) bildet, wobei der zumindest eine Bogen (VB1, VB2) zwei der Osteosyntheseplatten (O1a, O1b, O2a, O2b) miteinander verbindet.

5. Implantat (S) nach Anspruch 4, **dadurch gekennzeichnet, dass** der zumindest eine Bogen (VB1, VB2) einen inhomogenen Querschnitt aufweist, wobei der kleinste Querschnitt des zumindest einen Bogens (VB1, VB2) an einem Kontaktbereich (KB) zu den Osteosyntheseplatten (O1a, O1b, O2a, O2b) angeordnet ist.

6. Implantat (S) nach Anspruch 5, **dadurch gekennzeichnet, dass** der Kontaktbereich (KB) als Sollbruchstelle ausgebildet ist.

7. Implantat (S) nach einem der Ansprüche 4 bis 6 unter Rückbezug auf Anspruch 2 oder Anspruch 3, **dadurch gekennzeichnet, dass** das Verbindungselement (V) genau zwei lateral nach außen gewölbte Bögen (VB1, VB2) bildet, nämlich einen ersten Bogen (VB1) und einen zweiten Bogen (VB2),
- wobei der erste Bogen (VB1) eine der Osteosyntheseplatten (O1a) der ersten Osteosyntheseplattenanordnung (O1) mit einer der Osteosyntheseplatten (O2a) der zweiten Osteosyntheseplattenanordnung (O2) verbindet, und
- wobei der zweite Bogen (VB2) die andere Osteosyntheseplatte (O1b) der ersten Osteosyntheseplattenanordnung (O1) mit der anderen Osteosyntheseplatte (O2b) der zweiten Osteosyntheseplattenanordnung (O2) verbindet.

8. Implantat (S) nach Anspruch 7, **dadurch gekennzeichnet, dass** der erste Bogen (VB1) und der zweite Bogen (VB2) durch eine Verbindungsstrebe (VS) miteinander verbunden sind.

9. Implantat (S) nach einem der Ansprüche 1 bis 8, **dadurch gekennzeichnet, dass** das Verbindungselement (V) einen lateral hervorstehenden Vorsprung (VV) aufweist.

10. Implantat (S) nach Anspruch 9, **dadurch gekennzeichnet, dass** der Vorsprung (VV) als ringförmige Öse ausgebildet ist.

11. Implantat (S) nach Anspruch 9 oder Anspruch 10 unter Rückbezug auf Anspruch 8, wobei der Vorsprung (VV) an der Verbindungsstrebe (VS) ausgebildet ist.

12. Implantat (S) nach einem der Ansprüche 1 bis 11, **dadurch gekennzeichnet, dass** das Implantat (S) eine dritte Osteosyntheseplattenanordnung (O3) zur Verbindung des dritten Knochensegments (K3) mit einem vierten Knochensegment (K4) aufweist,
- wobei die dritte Osteosyntheseplattenanordnung (O3) genau eine oder genau zwei Osteosyntheseplatten (O3a, O3b) aufweist,
- wobei die Osteosyntheseplatte oder -platten (O3a, O3b) der dritten Osteosyntheseplattenanordnung (O3) eine stangenartige Form mit einem ersten Längsende und einem gegenüberliegenden zweiten Längsende aufweisen,
- wobei die Osteosyntheseplatte oder -platten (O2a, O2b) der zweiten Osteosyntheseplattenanordnung (O2) und die Osteosyntheseplatte oder -platten (O3a, O3b) der dritten Osteosyntheseplattenanordnung (O3) mit einem weiteren Verbindungselement (V2) unmittelbar verbunden sind,
- wobei das weitere Verbindungselement (V2) räumlich zwischen der zweiten und dritten Osteosyntheseplattenanordnung (O2, O3) angeordnet ist,
- wobei das weitere Verbindungselement (V2) derart ausgestaltet ist, dass das weitere Verbindungselement (V2) die zugeordneten Osteosyntheseplatten (O2a, O2b, O3a, O3b) an ihrem dem weiteren Verbindungselement (V2) zugewandten Längsende verbindet.

13. Implantat (S) nach Anspruch 12, **dadurch gekennzeichnet, dass** das weitere Verbindungselement (V2) analog zum Verbindungselement (V) gemäß einem der Ansprüche 4 bis 11 ausgebildet ist.

14. Implantat (S) nach einem der Ansprüche 1 bis 13, **dadurch gekennzeichnet, dass** das Implantat (S) einteilig ausgeführt und in einem additiven Verfahren gefertigt ist, beispielsweise in einem 3D-Druck-Verfahren, wobei das Implantat (S) patientenspezifisch geplant und geformt ist.

15. Implantat (S) nach einem der Ansprüche 1 bis 14, **dadurch gekennzeichnet, dass** das Implantat (S) ein Unterkiefer-Rekonstruktions-Implantat oder ein Oberkiefer-Rekonstruktions-Implantat ist.
